# EUROPEAN PATENT SPECIFICATION

(11) **EP 1 312 355 B1**
(45) Date of publication and mention of the grant of the patent: **11.06.2003**
(21) Application number: 01204432.7
(22) Date of filing: 20.11.2001
(51) Int. Cl.: A61K 9/00

(54) **Water-soluble non-effervescent pharmaceutical compositions comprising non-steroidal anti-inflammatory drugs**
Wasserlösliche pharmazeutische Darreichungsform, ausgenommen Brause-Formen, welche nicht-steroidale antientzündliche Wirkstoffe enthält
Non-effervescent composition pharmaceutique, soluble dans l'eau, contenant les Anti-Inflammatoires Non Steroidiens (AINS)

(43) Date of publication of application: 21.05.2003
(73) Proprietor: APR APPLIED PHARMA RESEARCH S.A., 6828 Balerna (CH)
(72) Inventor: Reiner, Alberto, 22100 Como (IT); Reiner, Giorgio, 22100 Como (IT)
(74) Representative: Pistolesi, Roberto

(56) References cited:
- EP-A- 0 418 043
- EP-A- 0 667 151
- EP-A- 0 945 132

## Description

The present invention relates to non-effervescent pharmaceutical compositions soluble in water and, in particular, to pharmaceutical compositions comprising a water-soluble salt of a non-steroidal anti-inflammatory drug (NSAID) in admixture with completely salified di- or tricarboxylic organic acids.

NSAIDs are drugs that have been used for long time in therapy, mainly due to their anti-inflammatory and analgesic properties. Structurally they pertain to some general classes, among which those of arylpropionic and arylacetic acid derivatives are herein particularly relevant. Examples of said acids are ibuprofen, keoprofen, naproxen, flurbiprofen and diclofenac (Merck Index, 12^{th} edition, 1996).

The oral formulations and, in particular, the drinkable aqueous solutions, of said NSAIDs could represent, in many cases, particularly advantageous means of administration, being easily ingested, painless and, due to the pre-dissolution of the active component, generally rapidly effective.

However the preparation of aqueous oral formulation of NSAIDs is affected by both the low solubility of the drug, especially in its non-salified form, and by the unpleasant and stinging taste given by said substances to the solution. This problem is particularly relevant for pediatric pharmaceutical formulations in which even the slightest residue of taste or irritation may imply a total refusal of the drug for the child.

In the literature, with the aim to obviate to that drawback, several effervescent and non-effervescent compositions have been studied, in which, besides solubilizing the drug, an attempt of masking the unpleasant taste by adding sweeteners, flavors or masking substances was made.

Effervescent formulations, such as those based on ibuprofen described in the patent US4689218, allow a rapid disintegration of the pharmaceutical form by using admixtures of carbonates and /or bicarbonates together with organic acids that, when placed in water, evolve carbon dioxide. In general said formulations assure a satisfying solubilization of the active compound while do not solve the problem of palatability: in fact, anyhow, their aqueous solutions show an unpleasant and stinging taste when ingested.

Among water-soluble non-effervescent NSAID formulations those described in the European patent applications EP906092 and EP418043 may be cited; the last one, in particular, describes compositions in which a water-soluble salt of ibuprofen is associated with a bicarbonate, a momohydrogenphosphate or a tribasic citrate with the aim to improve the low palatability in aqueous solution.

The document, which underlines the use of bicarbonates and reports that momohydrogenphosphates and tribasic citrates have a less effective masking activity, neither mentions other suitable organic acids nor, least of all, suggests the use of mixtures of said acids.

In addition EP418043 defines the use of additional excipients as optional, but solely exemplifies compositions which always include other taste-masking substances, such as sweeteners and flavors, in particular currant flavor, in addition to the masking agent above mentioned.

However the corresponding aqueous solutions, although containing flavors, still show a low palatability that makes their ingestion unpleasant. In addition the presence of flavors is not always ideal, especially for pediatric formulations. In fact, if the chosen flavor is not particularly pleasant to the child, it is in practice impossible to mask it by adding milk or other drinks, making the administration of the drug difficult. In this case, therefore, it would be preferable to have a sweetened but not flavored solution, in order to adapt it to the child taste with specific corrections.

In conclusion, as far as we know, the water-soluble NSAID compositions described in the art or available on the market do not satisfactorily solve the problem of palatability of NSAID aqueous solutions, notwithstanding the use of flavors.

We have now surprisingly found that it is possible to effectively mask the unpleasant taste of aqueous solutions of water-soluble NSAID salts without using flavoring substances, with obvious advantages in terms of acceptance by patients, simplification of the preparation process and reduction of costs for the final pharmaceutical form.

In the present contest the term "to mask" takes the meaning of significantly reduce or, preferably, eliminate the unpleasant feeling of irritation of the oral mucosa that usually follows the ingestion of classical NSAID aqueous solutions, comprising flavors and sweeteners.

Therefore, object of the present invention is a solid non-effervescent water-soluble pharmaceutical composition, in particular a granulate, comprising a water-soluble salt of a NSAID having an arylpropionic or arylacetic structure and a mixture of at least two pharmaceutically acceptable di- or tricarboxylic organic acids, completely salified, optionally in admixture with pharmaceutically acceptable excipients, in which said organic acids are present in amounts and proportions as to mask the taste of the salt of said NSAID in the aqueous solution.

A second object of the present invention is represented by palatable aqueous solutions obtainable by dissolving the above compositions in water; although the amount of water to be used is not binding, a solution according to the present invention is generally preparable by dissolving 900 to 3000 mg of composition, preferably 900 to 2000 mg, in 100-250 ml of water.

A third object of the invention relates to a process for manufacturing water-soluble granulates according to the invention.

Finally, a further object of the invention is represented by a method for masking the unpleasant taste of water-soluble NSAID salts in aqueous solution by dissolving a formulation according to the present invention in water.

The compositions according to the present invention comprise a water-soluble

NSAID salt having an arylpropionic or arylacetic structure. Non-limiting examples of said NSAIDs are ibuprofen, ketoprofen, naproxen, flurbiprofen, diclofenac, sulindac, fenoprofen, ibufenac, ketorolac, pranoprofen and aclofenac. Ibuprofen, ketoprofen, naproxen, flurbiprofen and diclofenac are preferred NSAIDs.

Generally, water-soluble salts of said NSAIDs usable in the present invention, include sodium or potassium salts or salts of pharmaceutically acceptable nitrogenous substances such as aminoacids, for example lysine or arginine, or aminoalcohols, such as for example methylglucamine. Said salts may be previously formed or, alternatively, directly prepared in situ according to techniques known to the skilled in the art.

The present compositions comprise a mixture of at least two pharmaceutically acceptable di- or tricarboxylic organic acids, completely salified, in addition to the water-soluble NSAID salt.

Organic acids suitable for the above use are, for example, dicarboxylic acids such as succinic, malic, fumaric, adipic and tartaric acid and the tricarboxylic acids such as citric acid, fumaric, tartaric and citric acid being particularly preferred.

However, apart from the above-mentioned acids, other pharmaceutically acceptable polycarboxylic organic acids can be successfully used which, suitably salified and dissolved in water, provide a solution having a pH from 7.2 to 8.5.

Usually said acids are used in the form of alkaline metal salts or as salts with pharmaceutically acceptable organic bases, such as for example, basic aminoacids, optionally as mixed salts, preferably as sodium or potassium salts.

In the compositions object of the present invention the above-defined salified organic acids are used in admixture, preferably as a binary mixture.

Preferred binary mixtures are citric acid / tartaric acid and citric acid / fumaric acid mixtures.

The organic acids composing the mixture can be salified with the same or different cations.

Said organic acids can be used in variable proportions, depending on the kind of NSAID chosen too and however such as to confer a good palatability to the final aqueous solution. In particular, in the case of binary mixtures, the weight ratio between the two salified acids is generally comprised between 15:1 and 0.07:1, preferably between 11:1 and 2:1; instead, the weight ratio between the total amount of said salified organic acids and the salified active compound is generally comprised between 1:1 and 10:1, preferably between 2:1 and 7:1.

In the preferred embodiment of the invention, the present formulation contains a tartaric acid / citric acid or fumaric acid / citric acid binary mixture, wherein the mutual weight ratio between the two above salified acids is comprised between 1:2 and 1:5 and, more preferably, said mixtures in which the weight ratio between the two above salified acids is comprised between 1:2 and 1:5 and in which the citric acid prevails.

With respect to the single dosage unit of the active drug the total amount of salified organic acids is generally comprised between 100 and 4000 mg, preferably between 150 and 2000 mg, wherein with the term "dosage unit" the amount of the active drug taken in a single administration is intended.

The optimization of the compositions in object, by selecting the kind of acid, their salts, optional sweeteners and the amounts thereof, falls within the normal capabilities of the skilled in the art and anyway is oriented to the achievement of the best possible technological result, that is palatability of the NSAID final aqueous solutions.

Said choice, obviously, takes also into account that the final pH of the aqueous solution can not reach extreme values in that it is known that, at acidic values, the acid form of the NSAID could precipitate while, at basic values, damaging effects on the mucosae could occur.

Generally, the pH of the aqueous solution deriving from the dissolution of the present compositions is comprised in the range from 7.0 to 8.5, preferably from 7.2 and 7.8, even more preferably at about 7.4.

It is useful to underline that the better palatability of the present compositions with respect to those described in the art is a consequence not only of a simple action of pH buffering of the final aqueous solution, but comes just from the presence of said particular completely salified organic acids in admixture, as experimentally proven in the comparative example n. 10.

In fact, in that experiment, a composition according to the present invention, even without flavors, results better than a known composition comprising tribasic citrate alone and having the same pH (example 8 of the patent EP418043) in the palatability test.

The non-effervescent water-soluble compositions of the present invention may be under different solid oral pharmaceutical forms, for example, in the form of a powder, a granulate or a chewable tablet. In addition liquid oral formulations, such as syrups or drops, obtainable by dissolving the above solid compositions or, alternatively, preparable by dissolving in water single pre-weighted components and, if necessary, by correcting the volume up to the desired value, are also within the scope of the invention.

A preferred composition, according to the present invention, is represented by granulates, in particular by a granulate comprising a mixture of sodium or potassium tartrate, tripotassium citrate and sodium or potassium diclofenac, in which said sodium or potassium tartrate and said tripotassium citrate are present in a weight ratio comprised from 1:1 to 2:1 and from 4:1 to 5:1, respectively, per unity of sodium or potassium diclofenac.

The compositions object of the present invention, with the aim to make the administration of the product even more pleasant, may comprise additional non flavoring pharmaceutically acceptable excipients, i.e. saccharin, aspartame, cyclamate, acesulfame, saccharose, sorbitol, fructose and mixtures thereof, in such amount as to appropriately sweet the final solution.

In addition, especially in the case of granulates and powders, it is possible to add suitable diluents such as mannitol, lactose, dextrose or other polyalcohols while, for chewable tablets, disintegrants or other excipients commonly used in the field may be included.

The preparation of the solid compositions in object, in case of granulates, preferably occurs as described in the experimental part while in case of powders and chewable tablets it is performed by mixing the components and, for tablets, by the subsequent compression of the mixture, according to traditional techniques. With a purely exemplifying and not limiting scope, some preparative examples of the compositions object of the present invention are now reported.

### Examples

Preparation of water-soluble granulates

The NSAID salt is accurately mixed with 50% of the total weight of the chosen di- and tricarboxylic acids and with the sweeteners. The mixture is kneaded with the lowest amount of water, it is granulated and it is dried in a ventilated oven. The so obtained granulate is directly mixed with the remaining 50% of the mixture of acids and, optionally, with the reported diluent in a granular form. Thus the water-soluble granulates of the following examples 1-9 were prepared:

### Example 1

| | | |
|---|---|---|
| Sodium Diclofenac | 50 mg | 25 mg |
| Dipotassium tartrate O.P. | 100 mg | 50 mg |
| Tripotassium citrate O.P. | 200 mg | 100 mg |
| Saccharin | 10 mg | 5 mg |
| Aspartame | 100 mg | 50 mg |
| Granular mannitol | 940 mg | 670 mg |
| Total | 1400 mg | 900 mg |

### Example 2

| | | |
|---|---|---|
| Potassium diclofenac | 50 mg | 25 mg |
| Disodium tartrate hydrate O.P. | 50 mg | 25 mg |
| Tripotassium citrate O.P. | 250 mg | 125 mg |
| Saccharin | 10 mg | 5 mg |
| Aspartame | 100 mg | 50 mg |
| Granular mannitol | 440 mg | 670 mg |
| Total | 900 mg | 900 mg |

### Example 3

| | | |
|---|---|---|
| Potassium diclofenac | 50 mg | 25 mg |
| Dipotassium tartrate O.P. | 200 mg | 100 mg |
| Trisodium citrate O.P. | 100 mg | 50 mg |
| Saccharin | 10 mg | 5 mg |
| Aspartame | 100 mg | 50 mg |
| Granular mannitol | 440 mg | 670 mg |
| Total | 900 mg | 900 mg |

### Example 4

| | |
|---|---|
| Sodium ibuprofen dihydrate | 256 mg |
| Disodium tartrate O.P. | 150 mg |
| Tripotassium citrate O.P. | 550 mg |
| Saccharin | 10 mg |
| Aspartame | 80 mg |
| Granular mannitol | 154 mg |
| Total | 1100 mg |

### Example 5

| | |
|---|---|
| Sodium ibuprofen dihydrate | 512 mg |
| Dipotassium tartrate O.P. | 100 mg |
| Tripotassium citrate O.P. | 1.100 mg |
| Saccharin | 30 mg |
| Aspartame | 100 mg |
| Granular mannitol | 158 mg |
| Total | 2000 mg |

### Example 6

| | |
|---|---|
| Ibuprofen lysine salt | 342 mg |
| Disodium tartrate O.P. | 100 mg |
| Tripotassium citrate O.P. | 450 mg |
| Saccharin | 10 mg |
| Aspartame | 80 mg |
| Granular mannitol | 118 mg |
| Total | 1100 mg |

### Example 7

| | |
|---|---|
| Sodium naproxen | 257 mg |
| Potassium fumarate O.P. | 100 mg |
| Tripotassium citrate O.P. | 500 mg |
| Saccharin | 10 mg |
| Aspartame | 80 mg |
| Granular mannitol | 135 mg |
| Total | 1100 mg |

### Example 8

| | |
|---|---|
| Sodium naproxen | 500 mg |
| Potassium tartrate O.P. | 350 mg |
| Tripotassium citrate O.P. | 800 mg |
| Saccharin | 20 mg |
| Aspartame | 160 mg |
| Granular mannitol | 170 mg |
| Total | 2000 mg |

### Example 9

| | |
|---|---|
| Ketoprofen lysine salt | 80 mg |
| Disodium tartrate O.P. | 100 mg |
| Tripotassium citrate O.P. | 250 mg |
| Saccharin | 10 mg |
| Aspartame | 100 mg |
| Granular mannitol | 360 mg |
| Total | 900 mg |

The compositions of the above reported examples 1-9, when dissolved in water, provide drinkable solutions characterized by an excellent palatability.

### Example 10 (comparative example)

In the present example the following compositions, according to EP418043 and to the invention, were prepared:

| | EP418043 (ex. 8) Invention | |
|---|---|---|
| Sodium ibuprofen | 512 mg | 512 mg |
| Disodium tartrate O.P. | ----- | 100 mg |
| Tripotassium citrate O.P. | ----- | 1100 mg |
| Trisodium citrate O.P. | 600 mg | ----- |
| Saccharin | 50 mg | 50 mg |
| Dextrose | 2000 mg | 1440 mg |
| Current flavor | 40 mg | ----- |
| Total | 3202 mg | 3202 mg |

The two compositions were dissolved in water (100 ml) providing clear solutions with a pH at about 7.4.

The composition according to the present invention, even without flavors, provides a perfectly drinkable solution and shows a greater palatability than the one known. In fact, after ingestion, it does not cause the unpleasant feeling of irritation that results with the comparison formulation.

## Claims

1. A water-soluble non-effervescent solid pharmaceutical composition comprising a water-soluble salt of a NSAID having an arylpropionic or arylacetic structure and a mixture of at least two pharmaceutically acceptable di- or tricarboxylic organic acids, completely salified, optionally in admixture with pharmaceutically acceptable excipients.

2. A composition according to claim 1 in which said NSAID is selected from ibuprofen, ketoprofen, naproxen, flurbiprofen and diclofenac.

3. A composition according to claim 1 in which said water-soluble salt of a NSAID is a sodium, potassium, lysine, arginine or metilglucamine salt.

4. A composition according to claim 1 in which said organic acid salts are salts of alkaline metals or pharmaceutically acceptable organic basis or mixtures thereof, preferably sodium or potassium salts.

5. A composition according to claim 1 in which said salified organic acids are present in an overall weight ratio comprised between 1:1 and 10:1 with respect to the salified NSAID, preferably between 2:1 and 7:1.

6. A composition according to claim 1 in which said salified organic acids are present in an overall amount between 100 and 4000 mg, preferably between 150 and 2000 mg, per dosage unit.

7. A composition according to claim 1 in which said mixture of di- or tricarboxylic organic acids comprises at least two acids selected among succinic, malic, fumaric, adipic, tartaric and citric acid.

8. A composition according to claim 7 in which said mixture of organic acids is a binary mixture wherein the weight ratio between the two organic acids is comprised between 15:1 and 0.07:1, preferably between 11:1 and 2:1.

9. A composition according to claim 7 in which said mixture of organic acids is a binary mixture of tartaric acid / citric acid or fumaric acid / citric acid in a reciprocal weight ratio comprised between 1:2 and 1:5.

10. A composition according to claim 1 which, by dissolution, gives a pH comprised between 7.2 and 7.8, preferably a pH of about 7.4, to the aqueous solution.

11. A composition according to claim 1 in which said excipients are sweeteners selected among saccharin, aspartame, cyclamate, acesulfame, saccharose, sorbitol, fructose and mixtures thereof.

12. A composition according to claim 1 in a granular form.

13. A composition in a granular form according to claim 12 comprising a mixture of sodium or potassium tartrate, tripotassium citrate and sodium or potassium diclofenac, in which said sodium or potassium tartrate and said tripotassium citrate are present in a weight ratio respectively comprised from 1:1 to 2:1 and from 4:1 to 5:1 per unity of sodium or potassium diclofenac.

14. A liquid oral pharmaceutical composition obtainable by dissolution in water of the composition according to claim 1.

15. A process for manufacturing a granulate according to claim 12 which comprises the steps of:
a) mixing the water-soluble salt of the NSAID, the optional sweeteners and 50% by weight of the organic acid mixture,
b) wet-granulating and drying the mixture, and
c) blending the granulate with the remaining 50% by weight of the organic acid mixture and, optionally, with a diluent in a granular form.

## Patentansprüche

1. Wasserlösliche, nicht-schäumende, feste pharmazeutische Zusammensetzung, die ein wasserlösliches Salz eines NSAID mit einer Arylpropionsäure oder Arylessigsäure-Struktur und eine Mischung von mindestens zwei pharmazeutisch akzeptablen, organischen Di- oder Tricarboxylsäuren, die vollständig in Salze überführt sind, umfaßt, wahlweise in Vermischung mit pharmazeutisch akzeptablen Arzneimittelträgern.

2. Zusammensetzung gemäß Anspruch 1, wobei der NSAID aus Ibuprofen, Ketoprofen, Naproxen, Flurbiprofen und Diclofenac ausgewählt ist.

3. Zusammensetzung gemäß Anspruch 1, wobei das wasserlösliche Salz eines NSAID ein Natrium-, Kalium-, Lysin-, Arginin- oder Methylglucamin-Salz ist.

4. Zusammensetzung gemäß Anspruch 1, wobei die organischen Säuresalze Salze von Alkalimetallen oder pharmazeutisch akzeptablen organischen Basen oder Mischungen davon sind, vorzugsweise Natrium- oder Kaliumsalze.

5. Zusammensetzung gemäß Anspruch 1, wobei die in Salze überführten organischen Säuren in einem Gesamt-Gewichtsverhältnis im Bereich zwischen 1:1 und 10:1 in Bezug auf das in Salz überführte NSAID vorliegen, vorzugsweise zwischen 2:1 und 7:1.

6. Zusammensetzung gemäß Anspruch 1, wobei die in Salze überführten organischen Säuren in einer Gesamtmenge zwischen 100 und 4.000 mg, vorzugsweise zwischen 150 und 2.000 mg pro Dosiseinheit vorliegen.

7. Zusammensetzung gemäß Anspruch 1, wobei die Mischung der organischen Di- oder Tricarboxylsäuren mindestens zwei Säuren umfassen, die unter Bernsteinsäure, Äpfelsäure, Fumarsäure, Adipinsäure, Weinsäure und Zitronensäure ausgewählt sind.

8. Zusammensetzung gemäß Anspruch 7, wobei die Mischung der organischen Säuren eine Binärmischung ist, worin das Gewichtsverhältnis zwischen den beiden organischen Säuren im Bereich zwischen 15:1 und 0,07:1, vorzugsweise zwischen 11:1 und 2:1 liegt.

9. Zusammensetzung gemäß Anspruch 7, wobei die Mischung der organischen Säuren eine Binärmischung von Weinsäure/Zitronensäure oder Fumarsäure/Zitronensäure in einem gegenseitigen Gewichtsverhältnis im Bereich zwischen 1:2 und 1:5 ist.

10. Zusammensetzung gemäß Anspruch 1, welche nach Auflösung der wässrigen Lösung einen pH im Bereich zwischen 7,2 und 7,8, vorzugsweise einen pH von etwa 7,4 verleiht.

11. Zusammensetzung gemäß Anspruch 1, wobei die Arzneimittelträger Süßstoffe sind, die unter Saccharin, Aspartam, Cyclamat, Acesulfam, Saccharose, Sorbitol, Fructose und Mischungen davon ausgewählt sind.

12. Zusammensetzung gemäß Anspruch 1 in granulärer Form.

13. Zusammensetzung in granulärer Form gemäß Anspruch 12, umfassend eine Mischung von Natrium- oder Kaliumtartrat, Trikaliumcitrat und Natrium- oder Kalium-Diclofenac, wobei das Natrium- oder Kaliumtartrat und das Trikaliumcitrat in einem Gewichtsverhältnis von jeweils im Bereich von 1:1 bis 2:1 und von 4:1 bis 5:1 pro Einheit des Natrium- oder Kalium-Diclofenacs vorliegt.

14. Flüssige orale pharmazeutische Zusammensetzung, die durch Auflösen der Zusammensetzung gemäß Anspruch 1 in Wasser erhältlich ist.

15. Verfahren zur Herstellung eines Granulats gemäß Anspruch 12, welches die Schritte umfaßt:
a) Mischen des wasserlöslichen Salzes des NSAID, des optionalen Süßstoffs und 50 Gew.-% der Mischung der organischen Säuren,
b) Naß-Granulieren und Trocknen der Mischung, und
c) Vermengen des Granulats mit den verbleibenden 50 Gew.-% der Mischung der organischen Säuren und wahlweise mit einem Streckmittel zu einer granulären Form.

## Revendications

1. Composition pharmaceutique soluble dans l'eau non effervescente solide comprenant un sel soluble dans l'eau d'un AINS ayant une structure arylpropionique ou arylacétique et un mélange d'au mois deux acides organiques dicarboxyliques ou tricarboxyliques pharmaceutiquement acceptables complètement salifiés, facultativement en mélange avec des excipients pharmaceutiquement acceptables.

2. Composition selon la revendication 1 dans laquelle ledit AINS est choisi parmi l'ibuprofène, le kétoprofène, le naproxène, le flurbiprofène et le diclofénac.

3. Composition selon la revendication 1 dans laquelle ledit sel soluble dans l'eau d'un AINS est un sel de sodium, un sel de potassium, un sel de lysine, un sel d'arginine ou un sel de méthylglucamine.

4. Composition selon la revendication 1 dans laquelle lesdits sels organiques sont des sels de métaux alcalins ou des bases organiques pharmaceutiquement acceptables ou leurs mélanges, de préférence des sels de sodium ou de potassium.

5. Composition selon la revendication 1 dans laquelle lesdits acides organiques salifiés sont présents selon un ratio en poids global compris entre 1:1 et 10:1 par rapport à l'AINS salifié, de préférence compris entre 2:1 et 7:1.

6. Composition selon la revendication 1 dans laquelle lesdits acides organiques salifiés sont présents en une quantité globale comprise entre 100 et 4 000 mg, de préférence entre 150 et 2 000 mg, par unité de dose.

7. Composition selon la revendication 1 dans laquelle ledit mélange d'acides dicarboxyliques ou tricarboxyliques comprend au moins deux acides choisis parmi l'acide succinique, malique, fumarique, adipique, tartarique et citrique.

8. Composition selon la revendication 7 dans laquelle ledit mélange d'acides organiques est un mélange binaire dans lequel le ratio en poids entre les deux acides organiques est compris entre 15:1 et 0,07:1, de préférence entre 11:1 et 2:1.

9. Composition selon la revendication 7 dans laquelle ledit mélange d'acides organiques est un mélange binaire d'acide tartarique / acide citrique ou d'acide fumarique / acide citrique selon un ratio en poids réciproque compris entre 1:2 et 1:5.

10. Composition selon la revendication 1 qui, par dissolution, donne un pH compris entre 7,2 et 7,8, de préférence un pH d'environ 7,4, à la solution aqueuse.

11. Composition selon la revendication 1 dans laquelle lesdits excipients sont des édulcorants choisis parmi la saccharine, l'aspartame, le cyclamate, l'acésulfame, le saccharose, le sorbitol, le fructose et leurs mélanges.

12. Composition selon la revendication 1, sous forme granuleuse.

13. Composition sous forme granuleuse selon la revendication 12 comprenant un mélange de tartrate de sodium et de potassium, de citrate de tripotassium et de sodium ou de diclofénac de potassium, dans lequel ledit tartrate de sodium ou de potassium et ledit citrate de tripotassium sont présents selon un ratio en poids compris respectivement entre 1:1 et 2:1 et entre 4:1 et 5:1 par unité de diclofénac de sodium ou de potassium.

14. Composition pharmaceutique orale liquide pouvant être obtenue par dissolution dans de l'eau de la composition de la revendication 1.

15. Procédé de fabrication d'un granulé selon la revendication 12 comprenant les étapes consistant à :
a) mélanger le sel soluble dans l'eau de l'AINS, les édulcorants facultatifs et 50 % en poids du mélange d'acide inorganique,
b) traiter le mélange par granulation humide et le sécher et
c) mélanger le granulé avec les 50 % en poids restant du mélange d'acide inorganique et, facultativement, avec un diluant sous forme granuleuse.
